# EUROPEAN PATENT APPLICATION

(11) **EP 2 477 392 A1**
(43) Date of publication of application: **18.07.2012**
(21) Application number: 10815206.7
(22) Date of filing: 02.07.2010
(51) Int. Cl.: H04N 5/335, A61B 1/04, G02B 7/02, G02B 23/26, H04N 5/225

(54) **IMAGE PICKUP DEVICE AND METHOD FOR MANUFACTURING IMAGE PICKUP DEVICE**

(30) Priority: 11.09.2009 JP 2009210848
(71) Applicant: Olympus Medical Systems Corp., Tokyo 151-0072 (JP)
(72) Inventor: HOSHI Kazuhisa, Tokyo 151-0072 (JP); ISHIDA, Yuya, Tokyo 151-0072 (JP); KUCHIMARU, Toru, Tokyo 151-0072 (JP); YAMASHITA, Tomoaki, Tokyo 151-0072 (JP); IWASAKI, Seiji, Tokyo 151-0072 (JP); MASAMORI, Ryosuke, Tokyo 151-0072 (JP); HIROYA, Jun, Tokyo 151-0072 (JP)
(74) Representative: Gunzelmann, Rainer
(86) International application number: PCT/JP2010/061337
(87) International publication number: WO 2011/030608

(57) **Abstract**

An image pickup apparatus includes a plurality of micro pins provided to project from a back surface in an image pickup device, a plurality of electric boards capable of mounting electric components and having board surfaces provided with through-holes or through-grooves formed to enable the plurality of micro pins to pass through respectively, and solder portions that fix the plurality of micro pins and the plurality of electric boards by soldering in land portions adjacent to the through-holes or the through grooves in a state in which the plurality of electric boards with the plurality of micro pins respectively passed through the through-holes or the through-grooves of the plurality of electric boards are stacked on the back surface of the image pickup device.

## Description

### Technical Field

The present invention relates to an image pickup apparatus including an image pickup device loaded on an endoscope or the like and a manufacturing method of the image pickup apparatus.

### Background Art

In recent years, endoscopes which have image pickup apparatuses loaded on distal end portions of insertion portions have come to be widely used in medical fields and industrial applications.

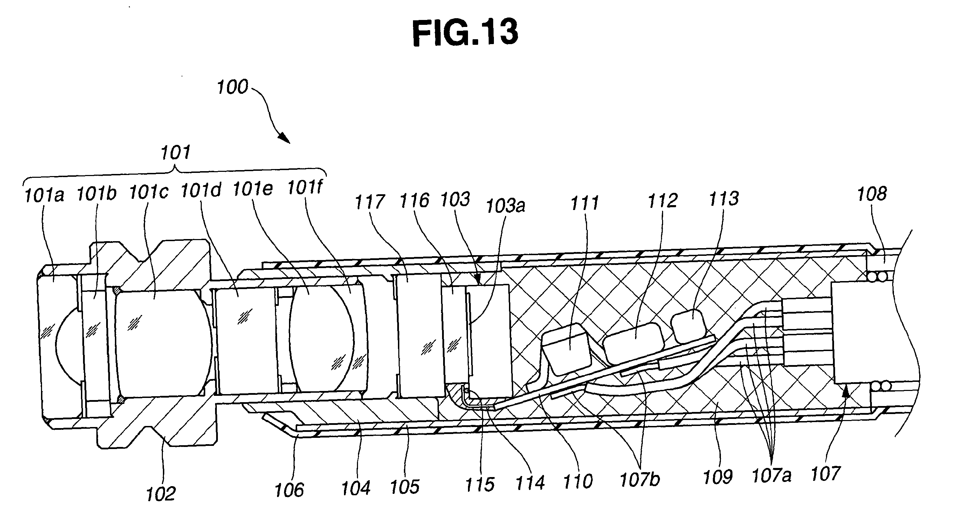

Fig. 13 shows a configuration of an image pickup apparatus 100 which is a conventional example similar to a configuration of an image pickup apparatus disclosed in Japanese Patent Application Laid-Open Publication No. 2008-177701, and is loaded on a distal end portion of an endoscope insertion portion.

In the image pickup apparatus 100, a main unit is configured by an objective lens system 101 configured by a plurality of lenses 101a to 101f, a lens frame 102 which holds the objective lens system 101, a device frame 104 which holds a part of an image pickup device 103, a shield member 105, a heat-shrinkable tube 106, a protective tube 108 with which a signal cable 107 is covered, and a sealing resin 109.

An outer peripheral surface of a rear end side of the lens frame 102 is fitted in an inner peripheral surface of a distal end side of the device frame 104, and a distal end side of the shield member 105 is fixed to an outer peripheral surface of a rear end side of the device frame 104. Further, the heat-shrinkable tube 106 with which outer peripheral surfaces of the device frame 104 and the shield member 105 are covered is fixed to the outer peripheral surface of the distal end side of the device frame 104. A rear end side of the heat-shrinkable tube 106 is fixed to an outer peripheral surface of a distal end side of the protective tube 108.

In the image pickup apparatus 100, an airtight space which is closed by the shield member 105 and the heat-shrinkable tube 106 is formed in a rear side along an optical axis of the objective lens group 101.

In the space, the image pickup device 103, a flexible printed board (abbreviated as FPC) 110 connected to the image pickup device 103, electronic components such as a transistor 111, a capacitor 112 and a resistor 113 which are loaded on the FPC 110, lead wires 107a which are extended from a signal cable 107 and the like are housed, and are sealed with the sealing resin 109 which is filled around the components.

Further, the image pickup device 103 is electrically connected to a lead wire 114 of the FPC 110 in a bonding portion 115.

Further, the FPC 110, a distal end side of which is connected to the image pickup device 103 by the lead wire 114, is extended from a bottom surface side of the image pickup device 103 to a diagonally upper direction at a back surface side thereof. The above described transistor 111 and the like are mounted on a top surface of the FPC 110 which is extended diagonally upward, and the lead wires 107a of the signal cable 107 are electrically connected to a bottom surface side at solder portions 107b.

An image pickup surface 103a of the image pickup device 103 is protected with a first cover glass 116, and a second cover glass 117 which is fixed to the device frame 104 is disposed at a front of the first cover glass 116.

Besides the endoscope including the image pickup apparatus 100 as shown in Fig. 13, for example, Japanese Patent Application Laid-Open Publication No. 2000-354584 discloses an image pickup apparatus including a CCD as an image pickup device which is adopted in a television camera fitted to an eyepiece portion of an optical endoscope.

In the image pickup apparatus, the CCD is attached to a seat, and a distal end side of an FPC is connected to a micro pin of the CCD, which projects to back surface sides of the CCD and the seat. The FPC is folded into a substantially U-shape and is extended to a rear side. Electronic components are mounted on the FPC.

In the image pickup apparatus 100 of the first conventional example of Fig. 13, a peripheral portion of the FPC 110 on which the electronic components are mounted in the back surface of the image pickup device 103 is of a structure which is extended to be long in the optical axis direction of the objective lens system 101. Therefore, when the image pickup apparatus 100 is provided at the distal end portion of the insertion portion of an endoscope in such a manner that the optical axis direction of the objective lens system 101 is parallel with the longitudinal direction of the distal end portion, the size in the longitudinal direction of the rigid distal end portion, that is, the rigid length becomes large.

When the size in the longitudinal direction of the rigid distal end portion is long, insertability into a bent body cavity and the like is reduced. Further, in the case of the image pickup apparatus of the second conventional example disclosed in Japanese Patent Application Laid-Open Publication No. 2000-354584, the U-shaped FPC is extended to the back surface side of the image pickup device.

Therefore, if the image pickup apparatus of the second conventional example is provided at the distal end portion of an endoscope, the disadvantage arises, that the size in the longitudinal direction of the distal end portion becomes large, as in the case of the image pickup apparatus 100 of Fig. 13.

Further, it is difficult to make the strength of the connection portion of the image pickup device 103 and the FPC 110 sufficiently large in the image pickup apparatus 100 of the first conventional example.

Further, when the first and the second image pickup apparatuses are provided in such a manner that the optical axis directions of the objective lens systems are in the directions orthogonal to the longitudinal directions of the distal end portions, the sizes in the radius directions of the distal end portions become large.

Further, in the above described image pickup apparatus of the second conventional example, the FPC loaded with the electronic components has the characteristic of being easily subjected to work such as bending, whereas much time and effort are likely to be required in assembly due to an adjusting operation or the like of setting the bent amount within a predetermined value so that the bent amount does not vary from one product to another.

Therefore, an image pickup apparatus which can make a size thereof small even when the image pickup apparatus is mounted on a distal end portion or the like of an insertion portion of an endoscope is desired, and an image pickup apparatus of a structure which can be easily assembled is desired.

The present invention is made in view of the above described problems, and has an object to provide an image pickup apparatus suitable for reduction in size and a manufacturing method of the image pickup apparatus.

Further, the present invention has another object to provide an image pickup apparatus which is suitable for reduction in size and is easily assembled, and a manufacturing method of the image pickup apparatus.

### Disclosure of Invention

### Means for Solving the Problem

An image pickup apparatus of the present invention includes a plurality of micro pins provided on a back surface of an image pickup surface in an image pickup device to project from the back surface,
a plurality of electric boards having board surfaces provided with through-holes or through-grooves formed to enable the plurality of micro pins to pass through, and
solder portions that fix the plurality of micro pins and the plurality of electric boards by soldering in land portions adjacent to the through-holes or the through-grooves in a state in which the plurality of electric boards with the plurality of micro pins passed through the through-holes or the through-grooves of the plurality of electric boards are stacked on a back surface of the image pickup device.

A manufacturing method of an image pickup apparatus including an image pickup device provided with a plurality of micro pins in the present invention includes
a first step of passing a plurality of micro pins provided to project from a back surface at an opposite side from an image pickup surface in the image pickup device through through-holes or through-grooves which are provided to pass through both board surfaces in a plurality of electric boards, and
a second step of fixing the plurality of micro pins and the plurality of electric boards at solder portions in a state in which the plurality of electric boards are stacked on the back surface of the image pickup device by melting solder balls provided at land portions adjacent to the through-holes or the through-grooves by heating the plurality of micro pins.

### Brief Description of the Drawings

Fig. 1 is a general configuration view of an endoscope system including a first embodiment of the present invention;
Fig. 2 is a sectional view showing a configuration of a distal end portion of an endoscope in the first embodiment of the present invention;
Fig. 3 is a sectional view showing a configuration of an image pickup apparatus of the first embodiment of the present invention;
Fig. 4 is a perspective view showing an image pickup device which is provided with micro pins and an electric board;
Fig. 5A is a view showing a first electric board;
Fig. 5B is a view showing a second electric board;
Fig. 5C is a view showing a third electric board;
Fig. 5D is a view showing a fourth electric board;
Fig. 6A is a perspective view showing a state in which the first electric board is brought into close contact with a back surface of the image pickup device;
Fig. 6B is a sectional view showing a state in which four electric boards are brought into close contact with the back surface of the image pickup device;
Fig. 6C is a sectional view showing a state in which solder portions are formed in land portions of each of the respective electric boards for the micro pins by using a heating furnace, in the state of Fig. 6B;
Fig. 7 is a flowchart showing a typical procedure of manufacturing the image pickup apparatus;
Fig. 8A is a perspective view showing a state in which an image pickup device and four electric boards in a first modified example are brought into close contact with one another;
Fig. 8B is an explanatory view of a case in which type discriminating micro pins are provided at the image pickup device;
Fig. 8C is a side view showing a state in which lead wires are electrically connected to micro pins which are projected from an image pickup device and four electric boards stacked on a back surface thereof in a second modified example;
Fig. 9A is a sectional view showing a configuration in which a signal cable is connected to a folded portion obtained by cutting out a flexible circuit board in the image pickup apparatus;
Fig. 9B is a view seen along an arrow A of Fig. 9A;
Fig. 10A is a view showing a configuration of a signal cable of a structure which enables a signal line group side and a sheath side outside the signal line group side to advance and retreat;
Fig. 10B is a view showing a state in which the signal line group of the signal cable of Fig. 10A is soldered to a board to be electrically connected to the board;
Fig. 10C is a view showing a state in which the sheath side which is outside the signal line group is moved after the soldering;
Fig. 11A is a view showing a configuration of the image pickup apparatus in which a signal cable set at a resin member is connected to pads provided at an end surface of a blocked mount component;
Fig. 11B is a view showing the resin member seen along an arrow B in Fig. 11 A;
Fig. 11C is an explanatory view of a process of manufacturing the resin member of Fig. 11B;
Fig. 11D is an explanatory view showing a process after the process of Fig. 11C;
Fig. 11E is an explanatory view of a product of a process of being grinded;
Fig. 11F is an explanatory view of the product in the case of being further grinded after the process shown in Fig. 11 E;
Fig. 12A is a perspective view showing the image pickup apparatus in which a signal cable is connected to pins of a blocked mount component;
Fig. 12B is a sectional view of a schematic configuration of the blocked mount component to which the signal cable is connected by soldering; and
Fig. 13 is a sectional view showing a configuration of an image pickup apparatus in a conventional example.

### Best Mode for Carrying Out the Invention

Hereinafter, embodiments of the present invention will be described with reference to the drawings.

### (First embodiment)

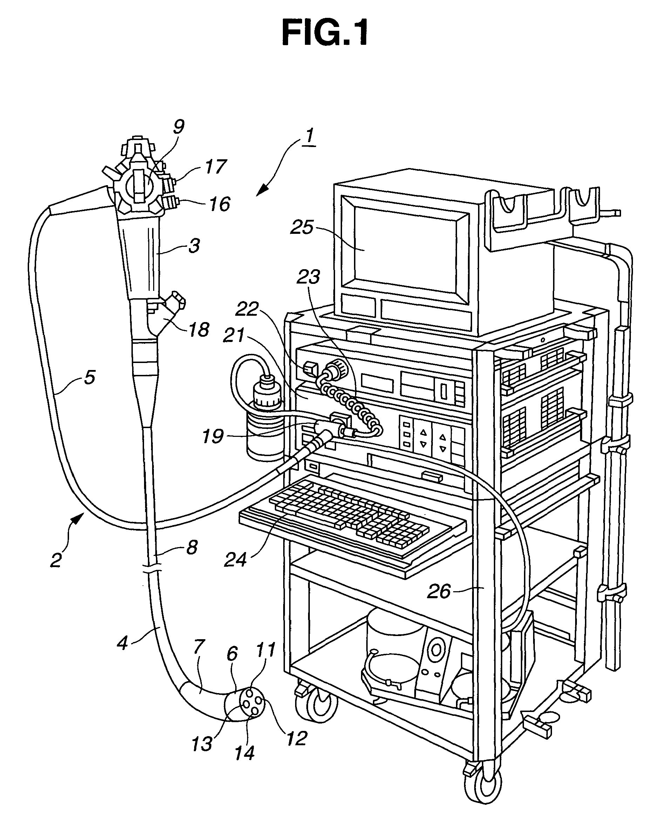

As shown in Fig. 1, an endoscope system 1 for performing endoscopy has an endoscope 2. The endoscope 2 is configured by an operation portion 3 which an operator grasps to perform an operation, an elongated insertion portion 4 which is formed at a front end of the operation portion 3 and is inserted into a body cavity or the like, and a universal cord 5 a proximal end of which is extended from a side portion of the operation portion 3.

Further, the insertion portion 4 includes a rigid distal end portion 6 which is provided at a distal end thereof, a bendable bending portion 7 which is provided at a rear end of the distal end portion 6, and a flexible tube portion 8 which is provided at a rear end of the bending portion 7, is long and has flexibility, and the bending portion 7 is capable of a bending operation by a bending operation lever 9 provided at the operation portion 3.

The distal end portion 6 of the insertion portion 4 is provided with an observation window at which an objective lens system 11 for performing optical observation is attached, a nozzle 12 which sprays a fluid such as water and air to a surface of the objective lens system 11, an illuminating window 13 which emits an illuminating light to illuminate, and a distal end opening 14 of a treatment instrument insertion hole.

In order to eject gas and a liquid selectively from the nozzle 12, a gas-supply and water-supply operation button 16, and a suction operation button 17 for selectively recovering mucus and the like in a body cavity from the distal end opening 14 of the treatment instrument insertion hole are placed at the operation portion 3. The treatment instrument insertion hole is formed by a tube or the like not illustrated which is placed in the insertion portion 4, and communicates with a treatment instrument insertion opening 18 provided in the vicinity of a front end of the operation portion 3.

Further, a connector 19 is provided at a tail end of the universal cord 5, and the connector 19 is connected to a light source device 21 of the endoscope. A base (not illustrated) to be a connection end portion of a fluid conduit which is projected from a distal end of the connector 19, and a light guide base (not illustrated) to be a supply end portion of an illuminating light are attachably and detachably connected to the light source device 21, and one end of a connection cable 23 is connected to an electric contact point portion provided on a side surface.

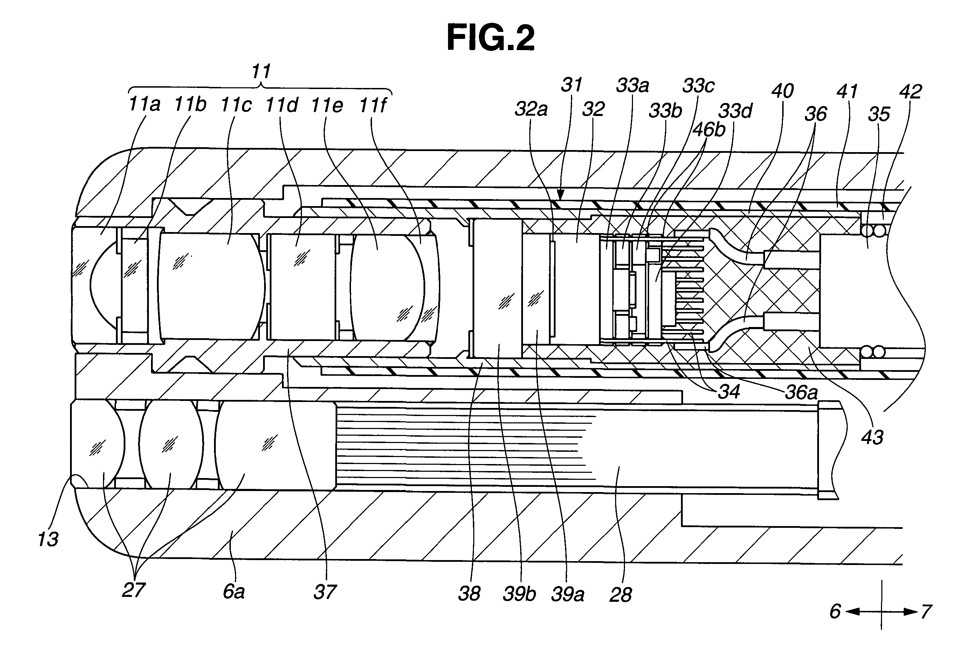

Further, a connector at the other end of the connection cable 23 is electrically connected to a video processor 22. The video processor 22 supplies a drive signal which drives an image pickup apparatus 31 (see Fig. 2) loaded on the distal end portion 6 of the endoscope 2, and performs signal processing for an image pickup signal (image signal) which is outputted from the image pickup apparatus 31 by supply of the drive signal to generate a video signal.

The video signal which is generated by the video processor 22 is outputted to a monitor 25 of the endoscope 2, and an image picked up by the image pickup apparatus 31 is displayed on a display surface of the monitor 25 as an endoscopic image. Peripheral devices such as the light source device 21, the video processor 22 and the monitor 25 are disposed on a rack 26 together with a keyboard 24 with which input and the like of patient information is performed.

The illuminating light which is generated in the light source device 21 is transmitted by a light guide 28 (see Fig. 2) which is inserted through the inside of the operation portion 3 and the insertion portion 4 from the universal cord 5, is expanded and emitted into a body cavity from the illuminating window 13 of the distal end portion 6 and can illuminate an object side of a diseased part or the like.

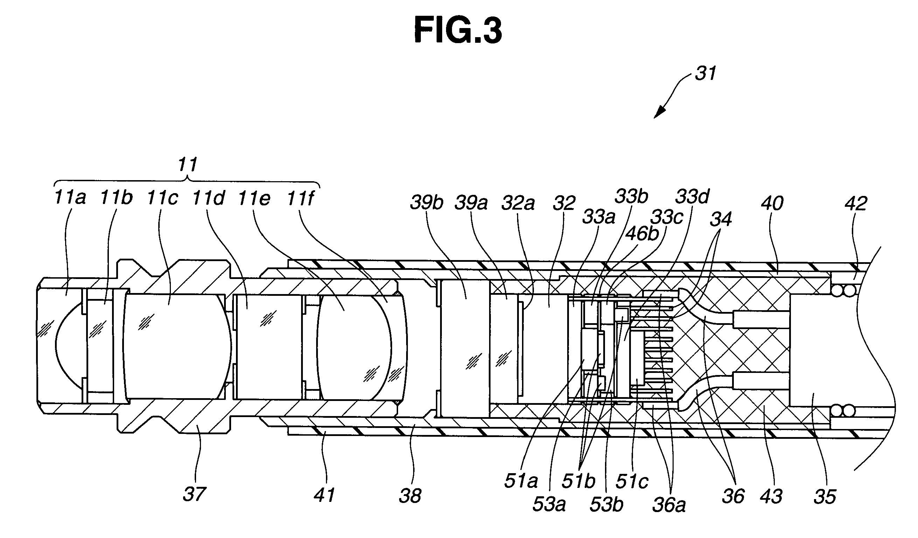

Fig. 2 shows a configuration of the distal end portion 6 which is provided with the image pickup apparatus 31 and the like, and Fig. 3 shows a configuration of the image pickup apparatus 31 region of Fig. 2.

A rigid distal end member 6a in a substantially columnar shape which configures the distal end portion 6 is provided with a through-hole which forms the observation window in a longitudinal direction of the distal end member 6a, and the image pickup apparatus 31 as image pickup means is attached in the through-hole.

Further, an illuminating lens system 27 and a distal end portion of the light guide 28 are attached in the illuminating window 13 adjacent to the observation window.

The above described image pickup apparatus 31 has the objective lens system 11 configured by a plurality of lenses 11 a to 11f, a solid image pickup device (abbreviated as an image pickup device) 32 including a charge coupled device (CCD) with an image pickup surface 32a which performs photoelectric conversion being disposed at an image formation position of the objective lens system 11, a MOS imager and the like, and a plurality of electric boards 33a, 33b, 33c and 33d disposed on a back surface of the image pickup device 32.

When the electric boards 33a to 33d are described by being distinguished from one another, the electric boards 33a to 33d are also called the first electric board 33a to the fourth electric board 33d.

The plurality of electric boards 33a, 33b, 33c and 33d are each soldered to a plurality of micro pins 34, ..., 34 in solder portions 46b respectively in a state in which the electric boards 33a, 33b, 33c and 33d are stacked with the plurality of micro pins 34, ..., 34 which are provided to project from a back surface of the image pickup device 32 in a direction orthogonal to the back surface passing through the electric boards 33a, 33b, 33c and 33d. Each of the micro pins 34 is formed of a metal wire such as a copper wire circular in section, for example.

Further, distal ends of lead wires 36 as a plurality of cables or signal lines which configure a signal cable 35 which transmits a drive signal and an image pickup signal are connected to distal ends of a plurality of micro pins 34, ..., 34 which pass through the plurality of stacked electric boards 33a, 33b, 33c and 33d to project to a rear side by soldering, and lead wire connection portions 36a (see Fig. 3) are formed.

As will be described later, a blocked mount component 51 a (the reference sign omitted in Fig. 2) and the like are mounted on one board surface in, for example, the electric board 33a in the plurality of electric boards 33a to 33d.

The electric board 33b is provided with an opening portion 53a which houses the blocked mount component 51a so that the electric board 33b can be stacked on the electric board 33a in layer. The other electric boards 33c and 33d are also configured to be easily stacked.

The plurality of lenses 11 a to 11f which configure the objective lens system 11 are fixed to a substantially cylindrical lens frame 37. A rear end side of the lens frame 37 is fitted in a front end side of a device frame 38 in which the image pickup device 32 is attached.

In the image pickup device 32, a first cover glass 39a is attached directly in front of the image pickup surface 32a to protect the image pickup surface 32a.

A second cover glass 39b to which a front surface of the first cover glass 39a is fixedly attached, and which is in a size larger than the size of the first cover glass 39a is fixedly attached to the device frame 38 by bonding or the like, and thereby the image pickup device 32 is attached to the device frame 38.

The lens frame 37 and the device frame 38 are fixed to each other in a state in which the optical image of an object by the object lens system 11 is focused onto the image pickup surface 32a to be formed on the image pickup surface 32a by adjustment of the focus. A shield frame 40 for electromagnetic shielding is placed at a rear end side of the device frame 38.

The device frame 38 and the shield frame 40 are covered with a heat-shrinkable tube 41. A rear end of the heat-shrinkable tube 41 is fixed to a distal end side of a protective tube 42 with which the signal cable 35 is covered.

Further, a sealing resin 43 is filled in a space around the image pickup device 32 disposed inside the shield frame 40, the plurality of stacked electric boards 33a to 33d, the lead wires 36 of the signal cable 35 and the like to seal the space (to prevent humidity and the like) and fix the image pickup device 32, the plurality of electric boards 33a to 33d and the like to the inside of the shield frame 40.

A region in the vicinity of the rear end of the sealing resin 43 in Fig. 2 is a rear end of the rigid distal end portion 6, and the bendable bending portion 7 is formed adjacently to the rear end.

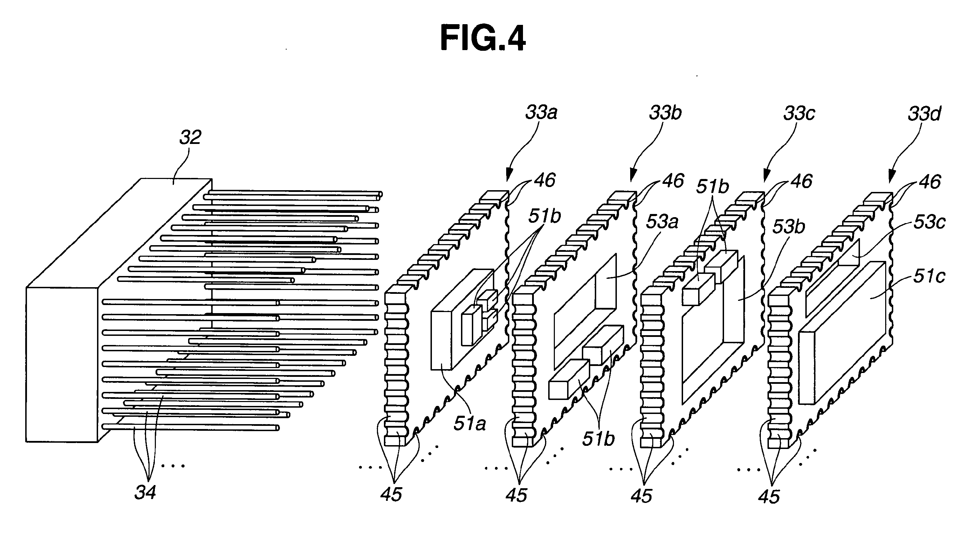

Fig. 4 shows the image pickup device 32 which is provided with the plurality of micro pins 34, ..., 34 on the back surface, and the plurality of electric boards 33a to 33d which are mechanically fixed and electrically connected to the plurality of micro pins 34, ..., 34 by soldering in the state before being assembled (attached).

As shown in Fig. 4, the image pickup device 32 is in a rectangular plate shape, and the plurality of micro pins 34, ..., 34 are arranged along the peripheral edge in the back surface thereof (along four sides in the illustrated example) to project at predetermined spaces, for example.

Meanwhile, each of the electric boards 33i (i=a, b, c, d) is in a rectangular plate shape in a size close to the size of the rectangle of the image pickup device 32, for example, and is provided with through-grooves 45, ..., 45 as through-passages which allow the plurality of micro pins 34, ..., 34 of the image pickup device 32 to pass through from one board surface to another board surface respectively.

The respective through-grooves 45 are formed in shapes close to semicircles, for example, at the same spaces as the spaces of the arrangement of the above described micro pins 34 on four end surfaces (more specifically, a top, a bottom, a left and a right end surfaces) of each of the electric boards 33i.

More specifically, in each of the electric boards 33i, a plurality of through-grooves 45, ..., 45 are provided at positions corresponding to the positions where the plurality of micro pins 34, ..., 34 are respectively provided in the image pickup device 32.

Further, the plurality of micro pins 34, ..., 34 are provided along the perimeter in the back surface of the image pickup device 32, and the plurality of through-grooves 45, ..., 45 which allow the plurality of micro pins 34, ..., 34 to pass through are provided along the perimeter of the board surface in each of the electric boards 33i, whereby the boards are structured to be easily stacked and arranged to be compact even when electronic components are mounted on the board surfaces as follows.

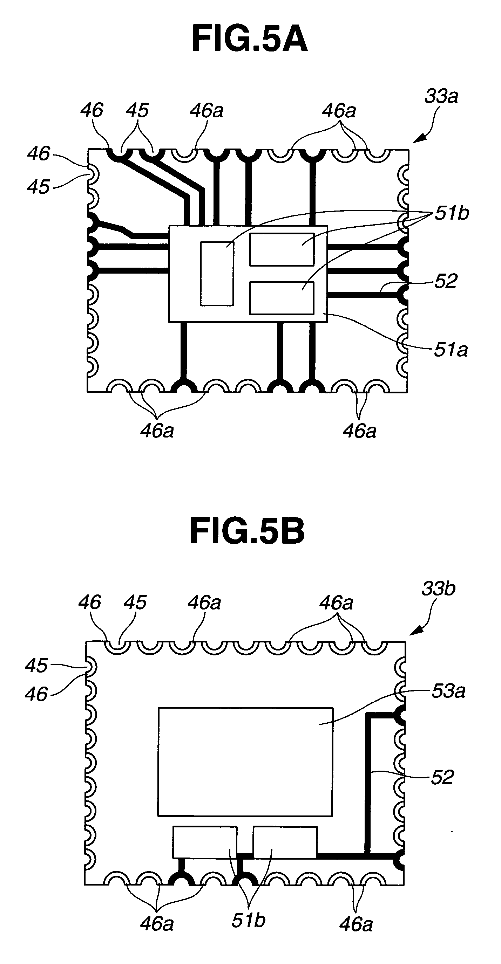

Figs. 5A to 5D respectively show the board surfaces at one side when the first electric board 33a to the fourth electric board 33d are seen from the right side of Fig. 4, that is, the board surfaces at the side opposite from the image pickup device 32 side.

As shown in Figs. 5A to 5D, land portions 46 which are respectively provided with solder balls are formed at semicircular ring portions adjacent to the respective through-grooves 45 in the board surfaces. On the board surfaces at the other side (that is, the board surfaces at the image pickup device side), the land portions 46 which are provided with solder balls adjacently to the through-grooves 45 are not provided.

The plurality of micro pins 34, ..., 34 which project from the back surface of the image pickup device 32 as shown in Fig. 4 can be sequentially passed through the through-grooves 45, ..., 45 in the electric boards 33a, 33b, 33c and 33d which are shown at the right side of the plurality of micro pins 34, ..., 34.

As shown in Fig. 5A, a blocked mount component 51 a as a mount component which is blocked is mounted, for example, in the vicinity of a center which is separated from the peripheral edge in which the through-grooves 45 are provided in the board surface at the side opposite from the image pickup device side in the electric board 33a. The blocked mount component 51a has electronic components 51b further mounted on a rectangular board.

The blocked mount component 51a is disposed so that the edges in the vertical direction are located at the positions equidistant from the two sides opposed in the vertical direction as shown in Fig. 5A in the electric board 33a. The blocked mount component 51 a is mounted so as to satisfy an equidistant relation with respect to a horizontal direction similarly to the case of the vertical direction (though the value of the distance differs from the value of the distance in the case of the vertical direction).

Further, a print pattern 52 for electrically connecting to a plurality of land portions 46 and the blocked mount component 51 a is formed on the board surface.

The land portions designated by reference signs 46a in Fig. 5A (ditto for Figs. 5B to 5D) represent the land portions where the micro pins 34 which pass through the insides of the through-grooves 45 inside the land portions are electrically connected to the signal cable 35 by soldering.

Accordingly, all of the plurality of micro pins 34, ..., 34 are not electrically connected to the lead wires 36 of the signal cable 35, but plurality of micro pins which are some of the entire micro pins 34 are used for electric connection with the signal cable 35. The number of the plurality of micro pins in this case is at least two for power supply and grounding, and two or more for drive of the image pickup device 32 and output, that is, four or more. Accordingly, the number of the micro pins which are used in electric connection and the micro pins which are not used in electric connection is at least five, and in the present embodiment, the number of micro pins which are not used in electric connection is made larger than the number of micro pins which are used in electric connection.

By a plurality of micro pins 34, ..., 34 which are not used in electric connection, the mechanical strength in the case of the plurality of stacked electric boards 33a to 33d being fixed onto the back surface of the image pickup device 32 can be enhanced.

In the present embodiment, the ratio of the number of micro pins 34 which are not used in electric connection with the signal cable 35 is much larger than the number of micro pins 34 which are used in electric connection (More specifically, about four times larger. However, the ratio is not limited to the value of four times larger.), and therefore, the mechanical strength can be significantly enhanced.

As above, in the present embodiment, the micro pins 34 which are not used in electric connection has the function of enhancing the mechanical strength at the time of the electric boards 33a to 33d being fixed by being soldered in the land portions 46 of the electric boards 33a to 33d.

The electric board 33b shown in Fig. 5B is provided with a rectangular opening portion 53a capable of housing the blocked mount component 51a correspondingly to the position where the blocked mount component 51 a of the above described electric board 33a is provided. Accordingly, the blocked mount component 51a is housed in the opening portion 53a, and thereby, the components can be stacked with high density.

Further, compact electronic components 51 b are mounted on the board surface of the electric board 33b adjacently to the opening portion 53a, and the electronic components 51b are electrically connected to the land portions 46 by the print pattern 52.

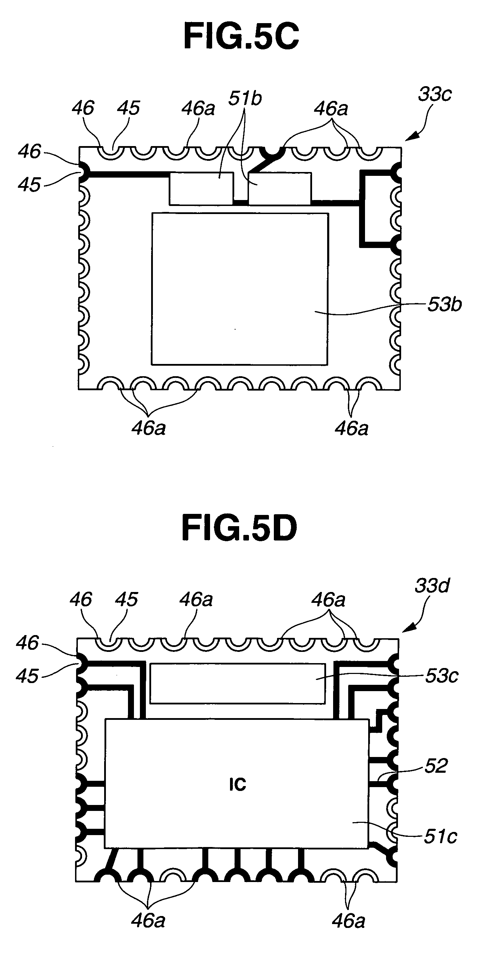

The electric board 33c shown in Fig. 5C is provided with a rectangular opening portion 53b which is capable of housing the blocked mount component 51a correspondingly to the position in which the blocked mount component 51a is provided, of the above described electric board 33a, and is capable of housing the electronic components 51b mounted on the electric board 33b.

Further, the compact electronic components 51 b are mounted on the board surface adjacently to the opening portion 53b, and the electronic components 5 1 b are electrically connected to the land portions 46 by the print pattern 52.

As shown in Fig. 5D, the electric board 33d which is stacked at the longest distance from the back surface of the image pickup device 32 is provided with a rectangular opening portion 53c which is laterally long and is capable of housing the electronic component 51b mounted on the above described electric board 33b. Further, an integrated circuit (IC) 51c as an electronic component the heating value of which is larger than the aforementioned electronic component 51 b, for example, is mounted in the vicinity of the center adjacent to the opening portion 53c on the board surface, and the IC 51 c is electrically connected to the land portions 46 by the print pattern 52. Instead of the IC 51c, an electronic component such as a resistor chip may be mounted.

For example, when the four electric boards 33a to 33d are stacked as shown in Fig. 4, as the electronic component 51b which is mounted on the electric board 33a which is separated from the end portions (more specifically, rear end portions) of the micro pins 34 by a large distance, the electronic component having heat resistance lower than that of the electronic components 51b which are mounted on the electric boards 33b and 33c separated by a shorter distance may be loaded.

The electronic component 51b and the like which are mounted on the plurality of electric boards 33a to 33d are selected and stacked like this, whereby when the end portions of the micro pins 34 are heated and soldered as will be described later, deterioration of the electronic components 51 b by heat can be reduced.

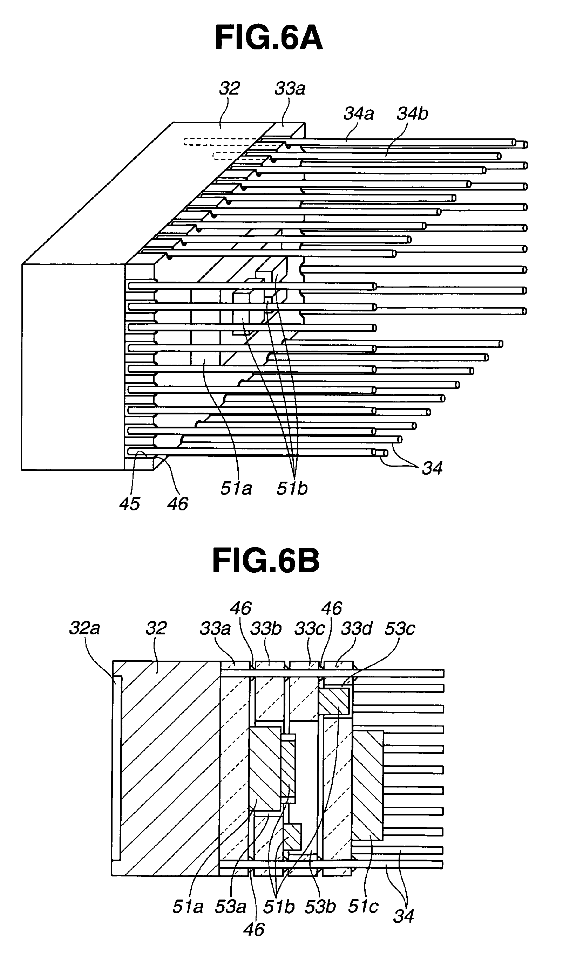

By adopting the structure of the electric boards 33a to 33d as shown in Fig. 4 or Figs. 5A to 5D, the four electric boards 33a, 33b, 33c and 33d can be set in a state in which the four electric boards 33a, 33b, 33c and 33d are stacked on the back surface of the image pickup device 32 to be closely contact with one another as shown in Fig. 6B.

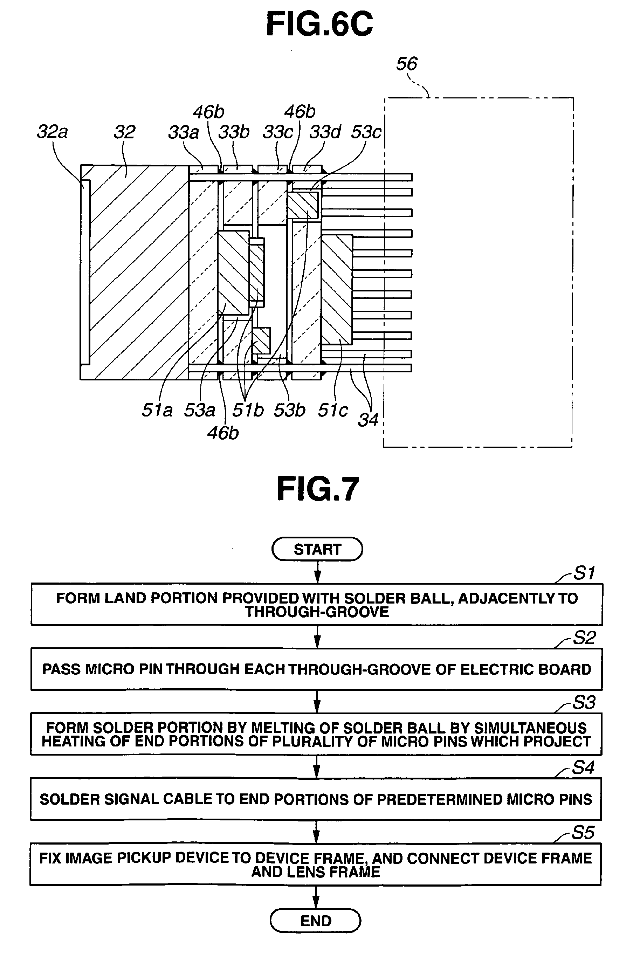

Further, as shown in Fig. 6C, the end portion sides of the plurality of micro pins 34, ..., 34 which project from the electric board 33d are heated in the stacked state, whereby each of the electric boards 33i and each of the micro pins 34 which pass through the through-grooves 45 can be fixed by the solder portions 46b by melting of the solder balls in the land portions 46. The land portion 46 and the micro pin 34 are fixed by soldering, and are electrically connected by conductive solder.

Fig. 6C shows the state in which the end portion sides of the plurality of micro pins 34, ..., 34 which project from the electric board 33d in Fig. 6B are heated by a heating furnace 56 which is schematically shown by the two-dot chain line, and the solder portions 46b are formed.

In this case, as shown in Fig. 6C, the solder portions 46b are formed by melting of the solder balls of the land portions 46 provided with the solder balls in each of the electric boards 33i, and therefore, are formed on only the board surface where the land portions 46 are formed, and which is on the side opposite from the image pickup device 32 side.

Accordingly, the amount of the heat which is applied to the image pickup device 32 at the time of fixing by soldering can be reduced more than in the case in which the land portions are provided on both the substrate surfaces in each of the electric boards 33i.

More specifically, until the solder balls melted by heating release heat to the surroundings and are fixed, part of the heat which the solder balls have is transmitted to the image pickup device 32 through the micro pins 34.

Therefore, the amount of heat which is exerted on the image pickup device 32 can be reduced more when the land portions provided with the solder balls are provided on the surface at only one side than when the land portions are provided on the board surfaces at both sides of the electric board 33i. Furthermore, the land portions 46 are provided on the board surface at the side opposite from the image pickup device side, and therefore, the amount of heat which is applied to the image pickup device 32 can be reduced more than when the land portions 46 are provided on the board surface at the image pickup device side.

Fig. 6A shows the state in which the first electric board 33a is brought into close contact with the back surface of the image pickup device 32 as the intermediate state of setting the state to the state shown in Fig. 6B from the state shown in Fig. 4.

Further, in Fig. 6A, the micro pin designated by reference sign 34a represents the micro pin which is electrically connected to an image pickup chip not illustrated inside the image pickup device 32, and a micro pin 34b which is provided adjacently to the micro pin 34a represents the micro pin 34b to be a dummy which has, for example, a shorter length in the image pickup device 32 than the length of the micro pin 34a, and is not electrically connected to the image pickup chip inside the image pickup device 32.

The length inside the image pickup device 32 of the micro pin 34b may be set to be substantially the same as in the case of the micro pin 34a. The micro pin 34b is not electrically connected to the image pickup chip as a matter of course.

Further, in the present embodiment, work of fixation and electric connection of the plurality of electric boards 33a to 33d and the plurality of micro pins 34, ..., 34 by soldering can be easily performed by the heating furnace as show in Fig. 6C.

Next, with reference to Fig. 7, a procedure of manufacturing the image pickup apparatus 31 will be described.

In the first step S1, a manufacturer forms the land portions 46 provided with the solder balls at the semicircular ring portions adjacent to the through-grooves 45, ..., 45 through which the plurality of micro pins 34, ..., 34 can pass, on the board surfaces at one side in the plurality of electric boards 33a to 33d which are incorporated into the image pickup apparatus 31, as shown in Figs. 5A to 5B.

In the next step S2, the manufacturer passes the plurality of micro pins 34, ..., 34 which project from the back surface of the image pickup device 32 as shown in Fig. 4 through the through-grooves 45, ..., 45 provided on the plurality of (more specifically, four) electric boards 33a to 33d.

Fig. 6A shows the state in which the plurality of micro pins 34, ..., 34 are passed through the through-grooves 45, ..., 45 of the first electric board 33a.

Subsequently, the four electric boards 33a to 33d are brought into the state of being stacked in close contact with one another on the back surface of the image pickup device 32. Fig. 6B shows the state. As shown in Fig. 6B, the plurality of electric boards 33a to 33d are stacked in layer, whereby the electronic components and the like can be three-dimensionally disposed on the back surface of the image pickup device 32 with high density.

In the next step S3, the manufacturer simultaneously heats the end portions of the plurality of micro pins 34, ..., 34 which project further from the electric board 33d by the heating furnace 56 as the heating device for heating shown in Fig. 6C.

Subsequently, the end portions of the plurality of micro pins 34, ..., 34 are simultaneously heated by the heating furnace 56, whereby the solder balls of the respective land portions 46 which the respective micro pins 34 pass through and are in contact with are melted.

After the end portions are heated until the solder balls of the land portion 46 are brought into the melted state, the image pickup device 32 is taken out from the heating furnace 56, and heating of the end portions of the plurality of micro pins 34, ..., 34 is stopped. One of the heating furnace 56 and the image pickup device 32 is moved, whereby heating and stop of heating may be performed. After heating is stopped, the end portions of the plurality of micro pins 34, ..., 34 may be cooled with a cooling device or the like. The melted solder balls are solidified, whereby the solder portions 46b are formed on the land portions 46. The solder portions 46c in Fig. 6C correspond to the case of the solidified state.

In the next step S4, the manufacturer connects the lead wires 36 of the signal cable 35 to a plurality of (for example, a fraction of the entire number of micro pins) micro pins 34, ..., 34 which should be actually connected in the plurality of micro pins 34, ..., 34 by soldering.

In the next step S5, the manufacturer attaches the cover glass 39b to a front of the cover glass 39a which protects the image pickup surface 32a of the image pickup device 32 by back adhesion or the like, and the cover glass 39b is fixed to the device frame 38 by adhesion or the like.

Further, the device frame 38 is fitted onto the lens frame 37 in which the objective lens system 11 is attached and focus is adjusted, after which, the device frame 38 and the lens frame 37 are connected and fixed. Further, the rear end portion of the device frame 38 is covered with the shield frame 40, and the sealing resin is filled therein. Subsequently, the heat-shrinkable tube 41 is attached to cover the device frame 38 and the shield frame 40, whereby the image pickup apparatus 31 shown in Fig. 3 is manufactured.

Further, the image pickup apparatus 31 is incorporated in the endoscope 2. In this case, the distal end side portion of the image pickup apparatus 31 is fixed to the through-hole of the distal end portion 6 of the endoscope 2 as shown in Fig. 2.

In the image pickup apparatus 31 of the present embodiment which is manufactured by the configuration and the manufacturing method as above, the plurality of electric boards 33a to 33d can be disposed compactly by being stacked on the back surface of the image pickup device 32, and the size thereof can be made small. More specifically, the image pickup apparatus 31 can be made compact.

Further, according to the image pickup apparatus 31, in the electric board adjacent to the electric board loaded with the electronic component on the board surface, the opening portion capable of housing the aforesaid electronic component is provided, and therefore, the electric board can be stacked with higher density than in the case in which the opening portion is not provided. More specifically, the image pickup apparatus 31 can be made compact.

Further, the solder portions 46b which make fixation by soldering are formed in the land portions 46 in the plurality of electric boards 33a to 33d with use of the plurality of micro pins 34, ..., 34.

In this case, in the present image pickup apparatus 31, the plurality of micro pins 34, ..., 34 which do not need to be electrically connected to the image pickup device 32 or the signal cable 35 are provided, and the micro pins 34, ..., 34 are also configured to be fixed by soldering in the land portions 46. Therefore, the mechanical strength of the image pickup apparatus 31 can be increased.

Further, fixation and electric connection of the image pickup device 32 and the plurality of electric boards 33a to 33d by soldering can be performed easily in a short time by simultaneous heating of the plurality of micro pins 34, ..., 34 by the heating furnace 56 or the like.

Further, the dummy micro pins (for example, reference sign 34b of Fig. 6A) which are not electrically connected to the image pickup chip inside the image pickup device 32 are provided. Accordingly, in the case in which the number of micro pins electrically connected to the image pickup chip of the inside needs to be increased for a different kind of image pickup device 32, such as the case in which the number of pixels of the image pickup device 32 is increased, the dummy micro pins can be used. More specifically, the present image pickup apparatus 31 is configured to be easily expanded.

Further, according to the image pickup apparatus 31, the land portions 46 are provided only on the board surfaces at the side opposite from the image pickup device side, and therefore, the influence which the heat by soldering has on the image pickup device 32 can be reduced.

Further, when the image pickup apparatus 31 is provided at the distal end portion 6 of the endoscope 2, the size of the rigid distal end portion 6, more specifically, the size in the longitudinal direction can be made small.

Further, in the present embodiment, a bendable flexible printed board on which electronic components are mounted is not used, and therefore, the time and effort are not required, which are required for adjustment and inspection so that the bending amount (bent shape) does not vary in each image pickup apparatus because the bending amount in the case of use of a flexible board is not fixed.

Therefore, assembly or manufacture of the image pickup apparatus 31 of the present embodiment can be performed easily in a short time. Further, variations among products can be decreased.

Further, when electronic components or the like are mounted and disposed on the back surface of the image pickup device 32 with use of the plurality of electric boards 33a to 33d, the electronic components can be disposed with heat resistance and the like of the electronic components and the like taken into consideration.

For example, the electronic component which easily generates heat (or generates heat) like the IC 51c can be disposed on the electric board 33d which is farthest from the image pickup device 32. The component which easily generates heat like this is mounted on the electric board which is separated from the image pickup device 32, whereby degradation of the characteristics or the like which is exerted on the image pickup device 32 (due to a temperature rise by the components easily generating heat) is reduced, and increase of noise of the image pickup device 32 due to a temperature rise can be reduced.

Further, the plurality of micro pins 34, ..., 34 are formed along the peripheral edge of the electric board, whereby the area in the case in which the electronic component and the like are mounted can be increased, and the degree of freedom in the case in which the electronic component is disposed can be increased. Further, the plurality of micro pins 34, ..., 34 are formed along the peripheral edge, and thereby, are given the function of protecting the plurality of electric boards stacked inside of the areas surrounded by the plurality of micro pins 34, ..., 34 as if the frame for reinforcement is disposed on the peripheral edge.

The micro pins 34 can be provided in the center instead of the peripheral edge, but providing the micro pins 34 in the center becomes the limitation in the case in which the electronic components are mounted.

In the present embodiment, the example in which the plurality of micro pins 34, ..., 34 are provided over the entire range of the peripheral edge in the back surface of the image pickup device 32 is shown, but the plurality of micro pins 34, ..., 34 may be provided in a part of the range along the peripheral edge.

For example, a plurality of micro pins 34, ..., 34 may be provided along the opposed two sides in the four sides shown in Fig. 4, or along the two sides adjacent in the orthogonal direction, that is, in an L-shape. Further, a plurality of micro pins 34, ..., 34 may be provided along one side or three sides.

Further, the through-grooves 45 which are provided in each of the electric boards 33i are not limited to the through-grooves 45 shown in Fig. 4 and the like, and may be provided to allow the micro pins 34, ..., 34 to pass through correspondingly to the plurality of micro pins 34, ..., 34 which are provided in the image pickup device 32.

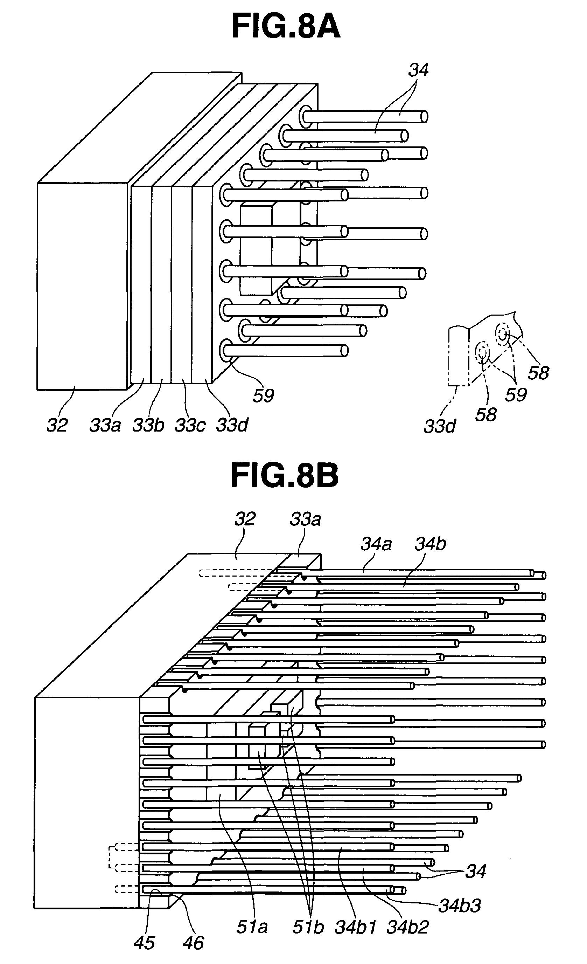

In the aforementioned embodiment, the through-grooves are not limited to the case of the through-grooves 45 which allow the plurality of micro pins 34, ..., 34 to pass through, but may be through-holes 58 as shown in Fig. 8A.

Fig. 8A shows the state in which the four electric boards 33a to 33d are stacked in layer on the back surface of the image pickup device 32, and the micro pin 34 passes through each of the through-holes 58.

Further, the two-dot chain line in Fig. 8A shows a part of the electric board 33d in the state in which the micro pins 34 are not passed therethrough, and the through-holes 58 are shown by the two-dot chain line. In this case, a land portion 59 in a circular shape provided with a solder ball is provided around the through-hole 58. Further, the number of the plurality of micro pins 34, ..., 34 is not limited to the numbers shown in Fig. 4 and Fig. 8A as long as the number of the micro pins 34, ..., 34 is not smaller than the number of the signal lines of the signal cable 35.

Further, the number of the plurality of electric boards which are stacked on the back surface of the image pickup device 32 is not limited to the case of four, and may be a plural number of two or more.

In Fig. 6A, the dummy micro pin 34b is shown, and the dummy micro pin 34b like this may be used for the micro pin for discriminating the type of the image pickup device 32. The application example is shown in Fig. 8B. Fig. 8B shows an example in which, for example, three micro pins in the plurality of dummy micro pins are set as type discriminating micro pins 34b1, 34b2 and 34b3 of the image pickup device 32 in the configuration of Fig. 6A. In the image pickup device 32 which is used in the image pickup apparatus 31 of the present embodiment, the type discriminating micro pins 34b1, 34b2 and 34b3 are in the state in which the respective type discriminating micro pins are electrically insulated, for example.

In the case of use of an image pickup device which has, for example, the number of pixels increased to be larger than the image pickup device 32, the type discriminating micro pins 34b1 and 34b2 are electrically connected in the image pickup device as shown by the two-dot chain line in Fig. 8B.

Accordingly, it is determined whether the type discriminating micro pins 34b1 and 34b2 are insulated or short-circuited, whereby the type of the image pickup device can be discriminated. By adoption of the three type discriminating micro pins 34b1, 34b2 and 34b3, the type discrimination function can be increased. The number of type discriminating micro pins is not limited to the case of two or three. Further, use of the type discriminating micro pins is not limited to the case of use of the type discriminating micro pins for discrimination of the type by setting the plurality of type discriminating micro pins in the short-circuited or insulated state, and the plurality of type discriminating micro pins may be connected with a resistor having a resistance value set for type discrimination. The type of the image pickup device may be enabled to be discriminated from measurement of the resistance value. When the resistance value is used, a plurality of kinds of resistance values are prepared, whereby the number of micro pins used for the type discriminating micro pins can be decreased.

The dummy micro pins are used for the type discriminating micro pins of the image pickup device like this, whereby the image pickup apparatus 31 is used more easily.

For example, when the image pickup apparatus is provided, which has the image pickup devices with the different numbers of pixels formed in the outer shapes of the same size as the kinds of image pickup devices, the image pickup apparatus is easily used for a wider use purpose, and the manufacture cost can be reduced. In such a case, the number of pixels of each of the image pickup devices can be discriminated more easily according to the type discriminating micro pins.

The number of type discriminating micro pins has to be a plural number. Further, when a plurality of type discriminating micro pins are provided, one of the plurality of type discriminating micro pins also can be used (that is, used for a double purpose) as the micro pin electrically connected to the image pickup chip inside the image pickup device 32, and also can be used for a double purpose as the micro pin connected to the signal cable 35.

For example, when the signal cable 35 and the four micro pins are connected, one of the micro pins also can be used as the type discriminating micro pin. The micro pin which is connected to the lead wire (signal line) for ground connection of the signal cable 35 also can be used as the type discriminating micro pin so that the influence can be reduced even the micro pin is used for the double purpose. When one of the micro pins is used for the double purpose like this, the type of the image pickup device can be discriminated with use of one more of the type discriminating micro pins. More specifically, the number of type discriminating micro pins can be reduced.

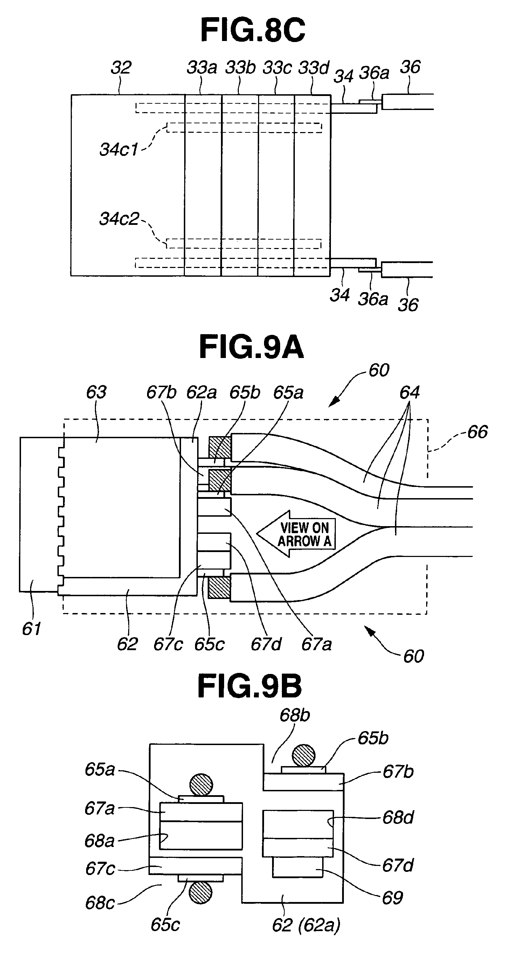

As a second modified example of the present embodiment, a configuration as shown in Fig. 8C may be adopted. In the aforementioned embodiment, each of the end portions of all the micro pins 34 which project from the back surface of the image pickup device 32 is configured to project from the board surface of the rear end side (abbreviated as the rear end surface) of the electric board 33d at the rearmost side which is the uppermost layer in the plurality of electric boards 33a to 33d stacked on the back surface of the image pickup device 32.

In contrast with this, in the case of the image pickup apparatus of the present modified example, in the case of the micro pins 34 which are electrically connected to the lead wires 36 (of the signal cable 35), each of the end portions of the micro pins 34 is configured to project from the rear end surface of the electric board 33d as shown in Fig. 8C, but in the case of micro pins 34c1 and 34c2 which are not electrically connected to the lead wires 36 (of the signal cable 35), each of end portions (which project to a rear side from the back surface of the image pickup device) in the micro pins 34c1 and 34c2 is configured not to project from at least the rear end surface of the electric board 33d.

In Fig. 8C, the two micro pins 34c1 and 34c2 are shown by the dotted lines, and the other micro pins which are not electrically connected to the lead wires 36 are also configured not to project from the rear end surface of the electric board 33d.

Further, Fig. 8C shows the case in which the positions of the respective end portions in the micro pins 34c1 and 34c2 differ from each other (that is, differ in length), but the micro pins 34c1 and 34c2 may be set at the same position (length). As in the case of the aforementioned embodiment, all the micro pins which project from the back surface of the image pickup device 32 are each formed by one pin.

With adoption of the configuration as above, when the lead wire 36 and the micro pin 34 are electrically connected by soldering and a lead wire connection portion 36a is formed in the present modified example, the micro pins 34c1, 34c2 and the like which are not used in (electric) connection do not project from the rear end surface of the electric board 33d, and therefore, are out of way when soldering is performed. Accordingly, the present modified example has the effect of facilitating soldering. The micro pins 34c 1 and 34c2 are not limited to the case of dummy micro pins, but can be the case of micro pins which are connected to the chip inside the image pickup device 32.

Incidentally, in recent years, miniaturization/increase in pixel rate of solid image pickup devices which are used in image pickup apparatuses has advanced. With this, high-frequency drive is required, as a result of which, the number of electronic components to be mounted in the image pickup apparatuses become large, the image pickup apparatuses become large in size, and problem arises, that the outer shapes of the distal end portions of endoscopes become large in size, or the rigid lengths become long.

For the above problem, the structure is adopted, which reduces the distance of soldering of the adjacent cables and prevents a short-circuit even if the distance is reduced, whereby an object to miniaturize the image pickup apparatus is realized. Figs. 9A and 9B show a configuration example of this case.

An image pickup apparatus 60 shown in Fig. 9A has a solid image pickup device (abbreviated as an image pickup device) 61, and in the image pickup device 61, an FPC 62 with a distal end being connected is extended from a region in the vicinity of a bottom surface of the image pickup device 61 to a rear side of a back surface of the image pickup device 61.

In the FPC 62 which is extended to the rear side of the image pickup device 61, a folded portion 62a which is folded into an L-shape to an upper side halfway is formed. A recessed portion is formed between the back surface of the image pickup device 61 and the folded FPC 62, and a blocked mount component 63 which is blocked is mounted therein. Further, cable solder portions 65a, 65b and 65c which connect a plurality of cables 64 which configure a signal cable by soldering are formed on the back surface of the folded portion 62a.

As shown by the dotted line of Fig. 9A, the structure is adopted, in which an adhesive 66 is filled around the blocked mount component 63 on the back surface of the image pickup device 61 and the cable solder portions 65a, 65b and 65c of the cables 64.

In this case, the cable solder portions 65a, 65b and 65c are provided at folded portions 67a, 67b and 67c which are each made by part of the FPC 62 being cut out and folded in (the folded portion 62a of) the FPC 62 as shown in Fig. 9B as a view taken along an arrow A of Fig. 9A.

Cutout portions 68a, 68b and 68c are cutout portions at the time of formation of the folded portions 67a, 67b and 67c respectively. In the cutout portion 68a and the like, the blocked mount component 63 is exposed.

The cable solder portion 65a of the folded portion 67a and the cable solder portion 65c of the folded portion 67c at a lower side of the cable solder portion 65a are formed to be close to each other in the vertical direction, but the folded portions 67a and 67c are interposed therebetween like walls, and therefore, the structure is provided, which hardly causes a short-circuit even though the distance between the adjacent cable solder portions 65a and 65c is small.

Further, the cable solder portion 65a of the folded portion 67a, and the cable solder portion 65b of the folded portion 67b are formed to be close to each other in the lateral direction, but are formed at positions at heights different stepwise in the vertical direction.

The folded portion 67b is in the state in which the folded portion 67b is interposed like a wall directly beside the cable solder portion 65a of the folded portion 67a, and therefore, the structure is provided, which hardly causes a short-circuit even though the distance between the adjacent cable solder portions 65a and 65b is small.

Further, the folded portion 67d which is formed by a cutout portion 68d which is obtained by the FPC 62 being cut out has an electronic component 69 mounted thereon.

The present image pickup apparatus 60 has the FPC 62 to which the image pickup device 61 is connected, and the blocked mount component 63 which is blocked and mounted, and the cable solder portions 65a and the like are provided in spaces obtained by the FPC 62 being cut out to have at least two cutouts and folded.

By adoption of the configuration as above, the structure can be provided, which interposes the folded board portions like walls, and hardly causes the adjacent cable solder portions 65a, 65b and 65c to short-circuit.

Further, the folded portions may be mounted with electronic components instead of being used for the cable solder portions, and in this case, the image pickup apparatus can be made compact.

Further, the mounted blocked mount component 63 is exposed at the cutout portion 68a, 68d and the like of the FPC 62 to allow the adhesive 66 to be filled therein. As a result, the state can be kept, in which the blocked mount component 63 is mounted without the FPC 62 being removed therefrom.

Consequently, according to the image pickup apparatus 60, a short-circuit in the adjacent cable solder portions is prevented, and miniaturization can be realized.

The conventional signal cable is provided with a general shield and an outer sheath on the outer periphery of the cable group with a plurality of cables being stranded. Therefore, when the cable portion is exposed from the end portion of the signal cable covered with the outer sheath, and is electrically connected to the board or the like which is connected to the image pickup device, the outer sheath at the end portion side of the signal cable needs to be removed (stripped) by a stripper.

In the conventional signal cable, when the stripper is applied to the signal cable to remove the outer sheath, the problem arises, that because of constraints in the structure of the stripper, the length by which the outer sheath is stripped from the signal cable, that is, the strip length becomes larger than an allowable length when miniaturization is to be realized.

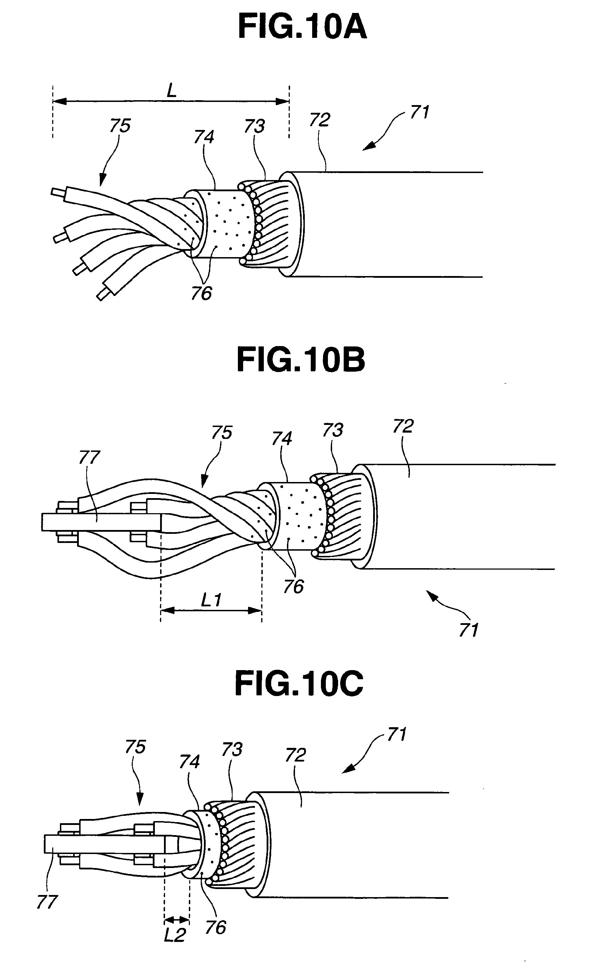

Therefore, in order that the strip length is made shorter even when the outer sheath is stripped by the stripper, and miniaturization is enabled to be realized, a configuration as shown in Fig. 10A may be adopted.

A signal cable 71 shown in Fig. 10A has an outer sheath 72, a general shield 73 inside the outer sheath, a sheath 74 inside the general shield, and a cable group 75 formed by a plurality of cables which are stranded inside the sheath 74, and a lubricant 76 is coated between an outer peripheral surface of the general shield 73 and an inner peripheral surface of the sheath 74, and between an outer peripheral surface of the cable group 75 and the inner peripheral surface of the sheath 74.

Fig. 10A shows a state in which the general shield 73 and the like inside the outer sheath 72 are formed from the state in which the outer sheath 72 is stripped by, for example, an existing stripper. Accordingly, a distance L in Fig. 10A corresponds to a strip length L.

The above described lubricant 76 is formed from a substance rich in lubricity (having a small friction coefficient), such as silicon particles, for example. Accordingly, by the lubricant 76, the general shield 73 side at the outer side, and the sheath 74 side at the inner side can be advance and retreat from each other in the longitudinal direction, and the cable group 75 and the sheath 74 side outside the cable group 75 are capable of advancing and retreating from each other in the longitudinal direction.

The case of connecting the cable group 75 to a board 77 which is connected to the image pickup device with use of the signal cable 71 of the configuration as above will be described. The outer sheath 72 is removed from the signal cable 71 by the existing stripper, and the cable group 75 side is moved to project from a distal end of the sheath 74 side outside the cable group 75, as shown in, for example, Fig. 10B.

The cable group 75 is projected from the distal end of the sheath 74, whereby the respective cables are soldered to the solder portions to which the cables should be connected in the board 77 to be electrically connected.

In this case, the distance between the rear end of the board 77 and the distal end of the sheath 74 is assumed to be L1.

After each of the cables is soldered, an operation of moving the outer sheath 72 side to the left side in Fig. 10B, that is, the board 77 side is performed, and thereby the distance between the rear end of the board 77 and the distal end of the sheath 74 can be made L2. In this case, the distances can be set to L1>L2. Further, the strip length in the case of Fig. 10C is shorter than L of Fig. 10A.

Accordingly, with use of the signal cable 71 of the structure like this, the strip length can be substantially made short.

Further, the length of the rigid portion which cannot be deformed at the distal end side of the signal cable 71 which is connected to the board 77 can be made short, and when the distal end side of the signal cable 71 connected to the board 77 configures an image pickup apparatus, the image pickup apparatus can be made compact by reduction in the strip length.

In the aforementioned description, the example in which the lubricant 76 is also provided on the outer peripheral surface of the sheath 74 is shown, but in the case of the configuration in which the lubricant 76 is provided only on the outer peripheral portion of the cable group 75, and the cable group 75 and the sheath side outside the cable group 75 are made capable of advancing and retreating, the strip length can be similarly made short.

Further, the projected amount of the cable group 75 side can be adjusted more than in the case of the conventional signal cable, and therefore, soldering to the land portions and the like of the board 77 can be performed by being set to the state in which soldering is more easily performed. Accordingly, the soldering work is facilitated.

Conventionally, when the signal cable including a plurality of cables is connected to a compact board configuring the image pickup apparatus which is loaded on the distal end portion or the like of an endoscope, the connection structure which is provided with pads aligned in a row in the axial direction of the board of the connection destination (longitudinal direction of the distal end portion) and the like have been adopted.

When the pads are aligned in the axial direction like this, the space where a plurality of cables are connected is small if the length of the depth of the board is not sufficient, and therefore, the problem arises, that the number of cables capable of being connected to the compact board becomes small.

Therefore, by adoption of the structure of collectively connecting the cables to the pads on the board surface or the like as will be described below, miniaturization of the cable connection portion and its peripheral portion in the image pickup apparatus, and assembly ease may be realized.

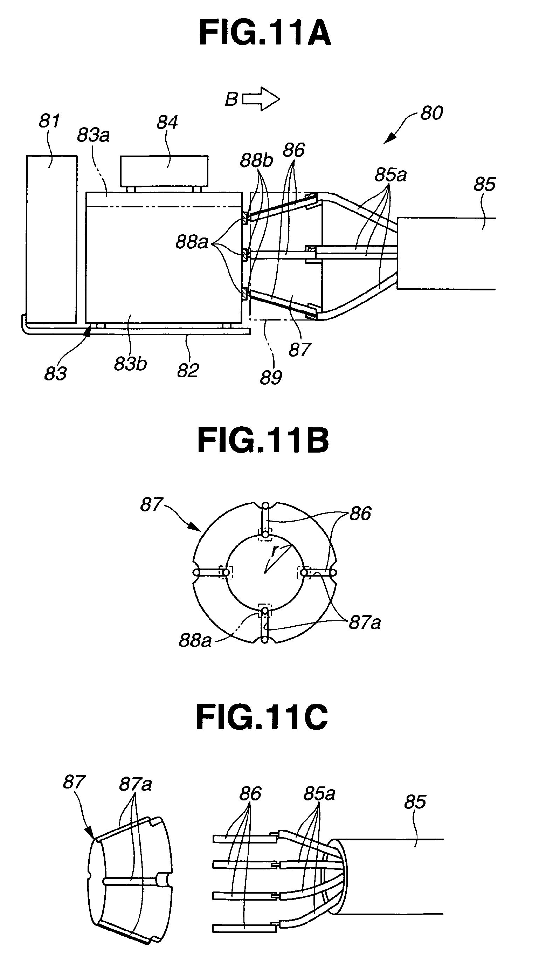

Fig. 11A shows a schematic configuration of an image pickup apparatus 80. The image pickup apparatus 80 has an image pickup device 81, an FPC 82 which is extended to a back surface side of the image pickup device 81 along, for example, a bottom surface of the image pickup device 81 with one end connected to the image pickup device 81, and a blocked mount component 83 which is mounted on a top surface of the FPC 82 extended to the back surface side of the image pickup device 81 and in which a plurality of mount components are arranged in one place.

The blocked mount component 83 has a top surface portion thereof formed by a ceramics board 83a, has mount components not illustrated mounted inside the blocked mount component 83, and is solidified into a cubic shape by a resin 83b, and a mount component 84 is mounted on a top surface of the blocked mount component 83.

Further, pads formed on a bottom surface of the blocked mount component 83 are electrically connected to the FPC 82 via the micro pins connected to ceramics board 83a and not illustrated.

A plurality of cables 85a which configure a signal cable 85 are electrically connected to a surface (rear end surface) at a rear end side in the blocked mount component 83 in a state in which the plurality of cables 85a are set in a resin member 87 in a truncated cone shape provided with grooves 87a (see Figs. 11B and 11C) which house pins 86 in a state in which the plurality of cables 85a are connected to the connecting pins 86, respectively.

In this case, pads 88a for wiring are formed at a plurality of equiangular spots (four spots in the illustrated example) on, for example, a predetermined radius r (see Fig. 11B in regard with the radius r) on a rear end surface of the blocked mount component 83, and each pad 88a is provided with a solder ball 88b.

The resin member 87 which is used in connection of the signal cable 85 is set to have a structure corresponding to the pads 88a at four spots provided on the rear end surface of the blocked mount component 83.

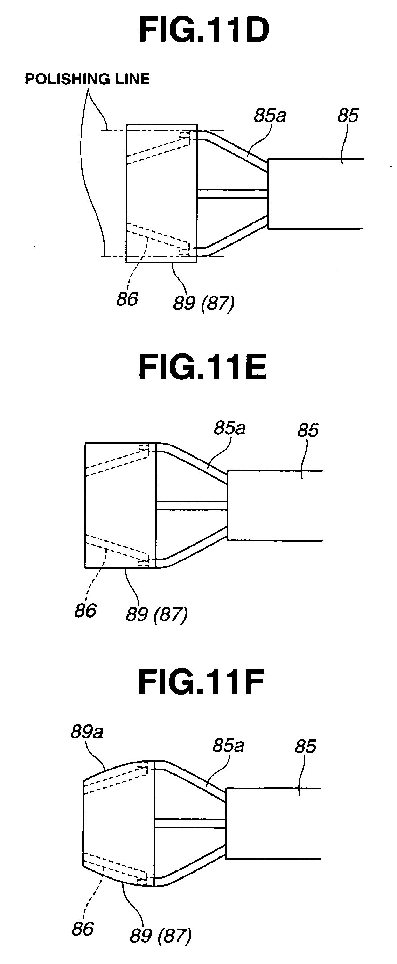

The two-dot chain line shown in Fig. 11 A shows an adhesive 89 for fixing the pins 86 in such a manner as to cover a periphery of the pins 86 after the pins 86 are housed in the grooves 87a of the resin member 87. The adhesive 89 will be described with Fig. 11D and the following drawings.

Fig. 11B shows a structure of the resin member 87 seen from a front side by view on arrow B of Fig. 11A. In this case, the positions of the pads 88a at the four spots in Fig. 11A are also shown by the two-dot chain lines.

As shown in Fig. 11B, the resin member 87 is in the truncated cone shape in which a distal end side to be a connection surface side to the pads 88a is in a tapering (converging) shape, the grooves 87a are formed at the four spots along the conical surface thereof (surface of the cone), and the pin 86 is housed in each of the grooves 87a.

A distal end of each of the housed pins 86 is set to project slightly in a distal end surface in a circular shape with a radius r and to be at a position opposed to each of the pads 88a.

Further, in a vicinity of a rear end of the groove 87a which houses each of the pins 86, the size of the groove 87a is made large, so as to be able to house a region in the vicinity of the solder portion 86a where the distal end of the cable 85a is soldered to the rear end of the pin 86.

Accordingly, the distal end of each of the pins 86 in the distal end surface of the resin member 87 shown in Fig. 11B is positioned to abut to the pad 88a, and each of the pins 86 is electrically connected to the pad 88a by soldering with use of a reflow furnace as a soldering device, whereby the image pickup apparatus 80 can be manufactured. The electric connection in this case can be easily performed with use of a reflow furnace.

The resin member 87 is used as above, whereby the cable 85a can be easily connected electrically to the pad 88a to which the cable 85a should be connected. Accordingly, assembly ease can be realized.

Further, with use of the resin member 87 and the pins 86 as shown in Fig. 11B, the distal ends of the pins 86 to be the electric connection portions with the pads 88a can be positioned to a small space portion (more specifically, the circle with the radius r). Accordingly, even when the pads 88a are provided in a small space, electric connection can be easily performed with use of the resin member 87, and the cable connection portion peripheral portion can be made compact.

Fig. 11C shows the state before the pins 86 are housed in the resin member 87, and a procedure of assembling the image pickup apparatus 80 will be described as follows.

As shown in Fig. 11C, the pins 86 are soldered to the cables 85a respectively, and the respective soldered pins 86 are housed in the grooves 87a of the resin member 87 which is shown at the left side thereof.

The resin member 87 which houses the pins 86 in the grooves 87a as described with Fig. 11C is covered with the adhesive 89 such as an epoxy resin as shown in Fig. 11D, and the pins 86 are fixed. The two-dot chain line in Fig. 11D shows one example of a grinding line.

The resin member 87 covered with the adhesive 89 shown in Fig. 11D is ground to the grinding line by a grind device, and the resin member 87 as shown in Fig. 11E is made.

Subsequently, with use of the resin member 87 shown in Fig. 11E, the distal ends of the pins 86 are positioned to the pads 88a and soldered to the pads 88a as described above, whereby the image pickup apparatus 80 is produced. In the resin member 87 shown in Fig. 11E, the outer peripheral side portions of the pins 86 may be further ground as shown in Fig. 11F, and ground surfaces 89a each in a conical surface shape, for example, may be formed.

In this case, the pins 86 are soldered to the pads 88a with use of the resin member 87 provided with the ground surfaces 89a.

The image pickup apparatus 80 has the blocked mount component 83 in which a plurality of mount components are arranged in one place, and the feature in which the wiring pads 88a are provided at the end surface of the aforesaid blocked mount component 83, the respective cables 85a which are electrically connected to the aforesaid blocked mount component 83 are connected to the pins 86, and the aforesaid pins 86 are electrically connected to the pads 88a of the blocked mount component 83.

Further, the aforesaid pins 86 are set at the resin member 87 so as to converge toward the blocked mount component 83. Further, the image pickup apparatus 80 has the feature in which the connection portions of the aforesaid pins 86 and the cables 85a are bonded and fixed by the resin member 87 or the like.

The outer periphery of the resin member such as the adhesive 89 which fixedly bonds the connection portions of the aforesaid pins 86 and the cables 85a may be shaved.

Conventionally, when a plurality of cables are connected to a compact board configuring an image pickup apparatus, the connection structure in which pads are provided at the stepped portion of the board of the connection destination, and the like are adopted. When a step is provided, and the cables are electrically connected to the step portion by soldering or the like, if the length in the height direction, especially, in the depth direction of the board is insufficient, the problem arises, that a space for connecting a plurality of cables becomes insufficient, and the number of cables which can be connected to the compact board becomes small.

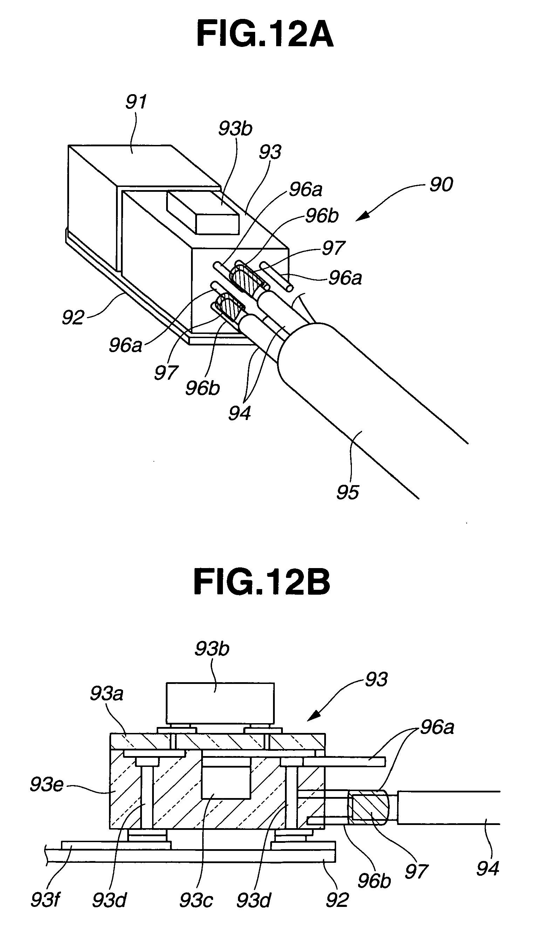

Therefore, in order to attain the object to provide a structure which enables miniaturization when the cables are connected to a compact board, a structure as shown in Figs. 12A and 12B may be adopted.

An image pickup apparatus 90 shown in Fig. 12A has an image pickup device 91, an FPC 92 connected to the image pickup device 91, a blocked mount component 93 which is mounted on the FPC 92 on a back surface side of the image pickup device 91 and in which a plurality of mount components are arranged in one place, and a signal cable 95 having a plurality of cables 94 which are electrically connected to the blocked mount component 93.

As shown in Fig. 12B, the blocked mount component 93 is in a rectangular parallel piped shape, and on a top surface of a ceramics board 93a forming a top surface of the blocked mount component 93, a first mount component 93b is mounted.

Further, a second mount component 93c is mounted on a back surface of the ceramics board 93a. Further, a plurality of micro pins 93d are provided to project downward from the back surface of the ceramics board 93a.

A resin 93e is filled in surroundings of the second mount component 93c and the micro pins 93d in the back surface of the ceramics board 93a to form the rectangular parallel piped shape.

Each of lower ends of the micro pins 93d is connected to the FPC 92 via an UBM (Under Bumping Metal) and a TAB (Tape Automated Bonding) tape 93f.

Further, the blocked mount component 93 is provided with conductive pins (first pins) 96a which are electrically connected to the micro pins 93d and the like configuring the blocked mount component 93 and use a metal material or the like, and pins (second pins) 96b which are not electrically connected to the blocked mount component 93.

The second pin 96b is a dummy pin which is not used for electric connection, and has a function of facilitating connection of the first pin 96a to be electrically connected and the cable 94 by soldering.

The second pin 96b is provided in such a manner that a proximal end thereof is buried in the resin 93e, for example, and is provided adjacently to a periphery of the first pin 96a.

The pins 96a and 96b are provided on a surface (rear surface) at an opposite side from the image pickup device 91 in the blocked mount component 93 as shown in Fig. 12A.

Subsequently, as shown in Fig. 12A, an end portion of the cable 94 is electrically connected by a solder portion 97 by solder in a state in which the end portion of the cable 94 is in contact with a plurality of adjacent pins, more specifically, the first pin 96a and the second pin 96b. Further, Fig. 12A also shows the state in which each of the cables 94 is electrically connected to the first pin 96a and the second pin 96b by soldering.

When the end portion of the cable 94 is connected to the first pin 96a, the end portion may be electrically connected by soldering by being brought into contact with the first pin 96a and one second pin 96b or more around the first pin 96a.

As above, in the present image pickup apparatus 90, the end portion of each of the cables 94 can be soldered by being brought into contact with the first pin 96a to which the end portion should be originally connected electrically, and the second pin 96b around the first pin 96a, and therefore, deposition of solder becomes better than in the case with only the first pin 96a, and soldering is facilitated. Consequently, according to the present image pickup apparatus 90, the cable 94 can be soldered by being brought into contact with two pins or more. Therefore, deposition of solder becomes favorable and soldering is facilitated.

Further, on the ground that soldering is easily performed, the cable 94 does not need to be led around to be connected by soldering in the case with only the first pin 96a, and the rigid length portion by the cable connection portion can be shortened. More specifically, by reduction of the rigid length of the cable connection portion, the image pickup apparatus 90 can be made compact.

Further, embodiments and the like which are configured by combination of parts of the aforementioned embodiments and the like also belong to the present invention.

This application is based upon and claims the benefit of priority from the Japanese Patent Application No. 2009-210848, filed on September 11, 2009; the entire contents of which are incorporated in the description, claims and drawings of the present application by reference.

## Claims

1. An image pickup apparatus, comprising:
a plurality of micro pins provided on a back surface of an image pickup surface in an image pickup device to project from the back surface;
a plurality of electric boards having board surfaces provided with through-holes or through-grooves formed to enable the plurality of micro pins to pass through; and
solder portions that fix the plurality of micro pins and the plurality of electric boards by soldering in land portions adjacent to the through-holes or the through-grooves in a state in which the plurality of electric boards with the plurality of micro pins passed through the through-holes or the through-grooves of the plurality of electric boards are stacked on the back surface of the image pickup device.

2. The image pickup apparatus according to claim 1,
wherein in at least one of the plurality of electric boards, a first electric board having a board surface on which an electronic component is mounted is included, and a second electric board stacked adjacently to the first electric board has an opening portion capable of housing the electronic component.

3. The image pickup apparatus according to claim 2,
wherein the plurality of micro pins which are provided to project from the back surface of the image pickup device are each formed by one pin.

4. The image pickup apparatus according to claim 2,
wherein the plurality of electric boards are in rectangular shapes in a substantially same size, and the electronic component which is mounted on the board surface is mounted in a center of the board surface.

5. The image pickup apparatus according to claim 2,
wherein the plurality of micro pins are provided along a peripheral edge on the back surface of the image pickup device.

6. The image pickup apparatus according to claim 2,
wherein the plurality of micro pins are provided along a peripheral edge along four sides of a rectangle in the rectangular back surface of the image pickup device.

7. The image pickup apparatus according to claim 2,
wherein respective end portions which project from the back surface of the image pickup device in at least four first micro pins or more in the plurality of micro pins are electrically connected to a signal cable which transmits a signal to and from the image pickup apparatus, and at least one second micro pin or more is or are not electrically connected to the signal cable.

8. The image pickup apparatus according to claim 2,
wherein respective end portions which project from the back surface of the image pickup device in at least four first micro pins or more in the plurality of micro pins are electrically connected to a signal cable which transmits a signal to and from the image pickup apparatus, and several times as many second micro pins as a number of the first micro pins are not electrically connected to the signal cable.

9. The image pickup apparatus according to claim 7,
wherein respective end portions which project from the back surface of the image pickup device, of the second micro pins which are not electrically connected to the signal cable, are disposed not to project to a rear side from at least a board surface at a rearmost side in the plurality of electric boards stacked on the back surface of the image pickup device.

10. The image pickup apparatus according to claim 8,
wherein respective end portions which project from the back surface of the image pickup device, of the second micro pins which are not electrically connected to the signal cable, are disposed not to project to a rear side from at least a board surface at a rearmost side in the plurality of electric boards stacked on the back surface of the image pickup device.

11. The image pickup apparatus according to claim 2,
wherein the land portions forming the solder portions are provided only on board surfaces at a side opposite from a side of the image pickup device in the plurality of electric boards.

12. The image pickup apparatus according to claim 2,
wherein the image pickup apparatus is loaded on a distal end portion of an insertion portion in an endoscope.

13. The image pickup apparatus according to claim 4,
wherein the image pickup apparatus is loaded on a distal end portion of an insertion portion in an endoscope.

14. The image pickup apparatus according to claim 2,
wherein respective end portions which project from the back surface of the image pickup device, of at least four first micro pins or more in the plurality of micro pins, are electrically connected to a signal cable which transmits a signal, and one second micro pin or more in the plurality of micro pins is or are used as a type discriminating micro pin or type discriminating micro pins of the image pickup device.

15. The image pickup apparatus according to claim 4,
wherein respective end portions which project from the back surface of the image pickup device, in at least four first micro pins or more in the plurality of micro pins, are electrically connected to a signal cable which transmits a signal, and one second micro pin or more in the plurality of micro pins is or are used as a type discriminating micro pin or type discriminating micro pins of the image pickup device.

16. The image pickup apparatus according to claim 15,
wherein one of the first micro pins and one of the second micro pins are used for common use.

17. The image pickup apparatus according to claim 4,
wherein on an electric board which is farthest from the back surface of the image pickup device in the plurality of electric boards, an integrated circuit is mounted.

18. The image pickup apparatus according to claim 8,
wherein a print pattern which connects the electronic component and at least one land portion to each other is formed on the board surface of the first electric board.

19. A manufacturing method of an image pickup apparatus including an image pickup device provided with a plurality of micro pins, comprising:
a first step of passing a plurality of micro pins provided to project from a back surface at an opposite side from an image pickup surface in the image pickup device through through-holes or through-grooves which are provided to pass through both board surfaces in a plurality of electric boards; and
a second step of fixing the plurality of micro pins and the plurality of electric boards at solder portions in a state in which the plurality of electric boards are stacked on the back surface of the image pickup device by melting solder balls provided at land portions adjacent to the through-holes or the through-grooves by heating the plurality of micro pins.

20. The manufacturing method of an image pickup apparatus according to claim 19, further comprising:
a third step of soldering only a plurality of first micro pins which correspond to a fraction of the plurality of micro pins in end portions of the plurality of micro pins which project from the back surface of the image pickup device to a plurality of lead wires configuring a signal cable.
